Europäisches Patentamt

European Patent Office

Office européen des brevets

(19)

(11) Numéro de publication: **0 128 618**
**B1**

(12) # FASCICULE DE BREVET EUROPÉEN

(45) Date de publication du fascicule du brevet:
**13.08.86**

(21) Numéro de dépôt: **84200776.7**

(22) Date de dépôt: **30.05.84**

(51) Int. Cl.⁴: **C 07 C 19/05, C 07 C 17/42**

(54) **Compositions stabilisées de 1,1,1-trichloroéthane.**

(30) Priorité: **09.06.83 FR 8309718**

(43) Date de publication de la demande:
**19.12.84 Bulletin 84/51**

(45) Mention de la délivrance du brevet:
**13.08.86 Bulletin 86/33**

(84) Etats contractants désignés:
**AT BE CH DE FR GB IT LI LU NL SE**

(56) Documents cité:
**EP-A-0 062 952**
**BE-A-858 017**
**FR-A-2 119 749**

(73) Titulaire: **SOLVAY & Cie (Société Anonyme), Rue du Prince Albert, 33, B-1050 Bruxelles (BE)**

(72) Inventeur: **Servais, Michel, Chaussée de Malines 315, B-1950 Kraainem (BE)**

Description

La présente invention concerne des compositions stabilisées de 1,1,1 - trichloroéthane, utilisables notamment pour le dégraissage des métaux et pour le nettoyage à sec des textiles.

On sait que le 1,1,1 - trichloroéthane, qui est utilisé notamment pour le dégraissage des métaux, le nettoyage à sec des textiles, et dans les aérosols, pose un problème de stabilité et de corrosion tout particulier. L'emploi, pour stabiliser le 1,1,1 - trichloroéthane, des stabilisants habituellement utilisés pour d'autres hydrocarbures chlorés ne donne pas, en général, des résultats satisfaisants. Ce phénomène a été attribué notamment au fait qu'il existe une grande réactivité entre le 1,1,1 - trichloroéthane et les métaux, tels que l'aluminium, le zinc et leurs alliages, et l'eau. Dès lors, on a développé des compositions stabilisées, spécifiques du 1,1,1 - trichloroéthane, nombreuses et variées. Parmi celles-ci on peut citer notamment des compositions ternaires à base d'époxydes, de nitroalcanes et de dioxanne décrites dans le brevet Etats-Unis 3 049 571 déposé le 18.3.1960 par The Dow Chemical Co. Toutefois, aucune des compositions proposées jusqu'à ce jour n'est vraiment efficace. En particulier aucune composition connue de 1,1,1 - trichloroéthane ne présente une stabilité satisfaisante dans des conditions extrêmement sévères telles que celles prévalant lors de la présence simultanée de métaux légers et d'eau.

Pour résoudre ce problème, la Demanderesse a déjà proposé dans la demande be brevet européen 82200418.0 du 2 avril 1982 des Compositions· stabilisées de 1,1,1 - trichloroéthane contenant un composé époxydé, un dérivé nitré et un éther interne de type furannique. Ces compositions, bien que donnant d'excellents résultats dans les conditions d'utilisation sévères, présentent lorsque la quantité d'eau est supérieure au taux de saturation en eau de la phase organique une difficulté dans la séparation des phases organique et aqueuse. C'est ainsi qu'on constate que la séparation complète par décantation de la phase aqueuse est excessivement lente.

La présente invention vise à remédier à cet inconvénient des compositions connues et à procurer des compositions stabilisées de 1,1,1 - trichloroéthane permettant une décantation parfaite et quasi instantanée de la phase aqueuse.

L'invention concerne à cet effet des compositions stabilisées de 1,1,1 - trichloroéthane contenant un composé époxydé, un composé nitré, un composé furannique et un ester d'alkyle dans lesquelles l'ester d'alkyle est l'acétate de méthyle.

Par composé époxydé, on entend les composés aliphatiques saturés ou insaturés contenant au moins un groupement époxyde dans leur molécule. De préférence, les compositions selon l'invention contiennent des composés aliphatiques saturés contenant de 3 à 6 atomes de carbone dans leur molécule tels que l'époxypropane, l'époxybutane, les 2 - méthylépoxypropanes, les 2 - méthylépoxybutanes, le glycidol et l'épichlorhydrine. De bons résultats ont été obtenus avec le 2 - méthyl - 2,3 - époxybutane et l'époxybutane.

La quantité totale de composé époxydé présente dans les compositions selon l'invention varie habituellement entre 0,01 et 50 grammes par litre. De préférence, cette quantité est comprise entre 0,1 et 20 grammes par litre. Tout particulièrement préférées sont des quantités comprises entre 1 et 10 grammes par litre.

Les composés nitrés présents dans les compositions selon l'invention sont de préférence des nitroalcanes contenant de 1 à 4 atomes de carbone dans leur molécule. De bons résultats ont été obtenus avec le nitrométhane, le nitroéthane et les nitropropanes 1 ou 2. Tout particulièrement préférés sont le nitroéthane et surtout le nitrométhane.

Les quantités de composés nitrés sont les mêmes que celles définies pour les composés époxydés.

Par composé furannique, on entend désigner le furanne ainsi que les dérivés du furanne où certains atomes d'hydrogène sont substitués par d'autres éléments ou groupements et plus particulièrement par des groupements aliphatiques saturés comprenant de 1 à 5 atomes de carbone.

Généralement, dans les dérivés substitués du furanne, le noyau furannique n'est substitué que par un ou deux groupements aliphatiques et les positions de substitution se situent habituellement sur les atomes de carbone 2 et 5 du noyau furannique.

Parmi les composés furanniques préférés figurent le 2 - méthylfuranne, le 2 - éthylfuranne, le 2 - isopropylfuranne, le 2 - propylfuranne et le 2,5 - diméthylfuranne. Les meilleurs résultats ont été obtenus avec le 2 - méthylfuranne qui est tout particulièrement préféré.

Les quantités de composé furannique présentes dans les compositions selon l'invention varient en général entre 0,1 et 100 gr par litre. De préférence, cette quantité est comprise entre 1 et 75 gr par litre. Tout particulièrement préférées sont des quantités comprises entre 5 et 50 gr par litre.

La quantité d'acétate de méthyle présente dans les compositions selon l'invention varie en général entre 0,01 gr et 50 gr par litre. De préférence cette quantité est comprise entre 0,1 et 20 gr par litre. Tout particulièrement préférées sont des quantités comprises entre 0,2 et 10 gr par litre.

Les compositions selon l'invention peuvent être utilisées dans toutes les applications requérant une bonne stabilité du 1,1,1 - trichloroéthane. Elles peuvent être utilisées dans des conditions sévères et en particulier en présence simultanée d'éléments métalliques notamment les métaux légers et d'eau.

Ainsi, les compositions selon l'invention conviennent particulièrement bien lorsque l'on opère en présence d'une quantité d'eau supérieure à la saturation en eau de la phase organique, c'est-à-dire lorsque l'on opère au contact d'une phase aqueuse nécessitant une décantation parfaite des deux phases organique et aqueuse.

Les compositions de l'invention présentent également une excellente aptitude au recyclage.

L'exemple qui suit n'a aucun caractère limitatif et montre la décantation améliorée de la phase organique de la phase aqueuse observée avec les compositions selon l'invention (essai 1) en comparaison avec une autre composition du même type (essai 2) ne contenant pas d'acétate de méthyle.

Essai 1

On introduit 500 cm³ de 1,1,1 - trichloroéthane, stabilisé au moyen de 4 gr/l de 1,2 - époxybutane, 5 gr/l de nitrométhane et 30 gr/l de 2 - méthylfuranne, et 2 gr/l d'acétate de méthyle dans un ballon de 1000 cm³ équipé d'un réfrigérant et raccordé à une source de vapeur d'eau.

La vapeur d'eau à 110°C est ensuite introduite dans le fond du ballon et le mélange est distillé par entraînement à la vapeur d'eau jusqu'à récupération de 99,3% de la quantité de 1,1,1 - trichloroéthane mis en oeuvre.

On laisse décanter le distillat et on récupère 100 cm³ de 1,1,1 - trichloroéthane stabilisé dans une éprouvette à pied.

On obtient instantanément une phase aqueuse bien décantée de la phase organique et bien limpide.

Essai 2

On opère selon le mode opératoire de l'essai 1 mais en mettant en oeuvre du 1,1,1 - trichloroéthane stabilisé au moyen de 4 gr/l d'époxybutane, 5 gr/l de nitrométhane et 30 gr/l de 2 - méthylfuranne.

Comme à l'essai 1, on obtient une phase aqueuse bien décantée de la phase organique mais il faut une heure pour que la phase aqueuse devienne limpide.

Revendications

1. Compositions stabilisées de 1,1,1 - trichloroéthane contenant un composé époxydé, un composé nitré, un composé furannique et un ester d'alkyle caractérisées en ce que l'ester d'alkyle est l'acétate de méthyle.

2. Compositions selon la revendication 1 caractérisée en ce que l'acétate de méthyle est présent dans des quantités comprises entre 0,1 et 20 gr par litre.

3. Compositions selon la revendication 1 ou 2 caractérisées en ce que le composé furannique est le 2 - méthylfuranne, le 2 - éthylfuranne, le 2 - isopropylfuranne, le 2 - propylfuranne ou le 2,5 - diméthylfuranne.

4. Compositions selon la revendication 3 caractérisées en ce que le composé furannique est le 2 - méthylfuranne.

Patentansprüche

1. Stabilisierte Zusammensetzungen von 1,1,1 - Trichlorethan enthaltend eine Epoxyd-Verbindung, eine Nitro-Verbindung, eine Furan-Verbindung und einen Alkylester, dadurch gekennzeichnet, daß der Alkylester Methylacetat ist.

2. Zusammensetzungen nach Anspruch 1, dadurch gekennzeichnet, daß das Methylacetat in Mengen zwischen 0,1 und 20 g pro Liter vorhanden ist.

3. Zusammensetzungen nach Anspruch 1 oder 2, dadurch gekennzeichnet, daß die Furan-Verbindung 2 - Methylfuran, 2 - Ethylfuran, 2 - Isopropylfuran, 2 - Propylfuran oder 2,5 - Dimethylfuran ist.

4. Zusammensetzungen nach Anspruch 3, dadurch gekennzeichnet, daß die Furan-Verbindung 2 - Methylfuran ist.

Claims

1. Stabilized 1,1,1 - trichloroethane compositions containing an epoxide compound, a nitro compound, a furan compound and an alkyl ester, characterized in that the alkyl ester is methyl acetate.

2. Compositions according to Claim 1, characterized in that the methyl acetate is present in quantities of between 0.1 and 20 grams per litre.

3. Compositions according to Claim 1 or 2, characterized in that the furan compound is 2 - methylfuran, 2 - ethylfuran, 2 - isopropylfuran, 2 - propylfuran or 2,5 - dimethylfuran.

4. Compositions according to Claim 3, characterized in that the furan compound is 2 - methylfuran.